# EUROPEAN PATENT SPECIFICATION

(11) **EP 1 853 739 B1**
(45) Date of publication and mention of the grant of the patent: **01.07.2009**
(21) Application number: 05748019.6
(22) Date of filing: 04.03.2005
(51) Int. Cl.: C14C 9/00, C07C 323/00

(54) **FUNGICIDAL COMPOSITIONS AND METHODS USING CYANODITHIOCARBIMATES**
FUNGIZIDE MITTEL UND VERFAHREN MIT CYANODITHIOCARBAMATEN
COMPOSITIONS FONGICIDES ET METHODES D UTILISATION DE CYANODITHIOCARBIMATES

(43) Date of publication of application: 14.11.2007
(73) Proprietor: BUCKMAN LABORATORIES INTERNATIONAL, INC., Memphis, Tennessee 38108 (US)
(72) Inventor: FENYES, Joseph, G., E., Germantown, TN 38138 (US); MCNEEL, Thomas, E., Memphis, TN 38134 (US); BRYANT, Stephen, D., Memphis, TN 38135 (US); WHITTEMORE, Marilyn, S., Germantown, TN 38139 (US); CSUROS, Zoltan, G., Memphis, TN 38119 (US); ZOLLINGER, Mark, L., Memphis, TN 38119 (US); MILLER, Robert, H., Eads, TN 38028 (US); MIGUEL, Naim, B., 75503-970 Itumbiaria, GO (BR)
(74) Representative: Matthews, Derek Peter
(86) International application number: PCT/US2005/006825
(87) International publication number: WO 2006/096159

(56) References cited:
- WO-A-20/04011682
- DD-A1- 275 433
- US-A- 3 299 129
- FOELDENY, R.: MONATSHEFTE FUR CHEMIE, vol. 126, 1995, pages 1035-1044, XP009057151

## Description

### Field of the Invention

The invention relates to using cyanodithiocarbimates to control fungal growth on animal hides during a hide tanning process. More particularly, the invention relates to the protection of wet blue hides from fungal attack and degradation.

### Background of the Invention

Leather, as is well known, is produced from animal hides through a multi-stage process which begins with the tanning of hides. Hides are a complex combination of fats and proteins (elastin, collagen, keratin) which provide for the structure and flexibility of the hide. A leather tanning process involves large shifts in temperature, pH, and types of chemistry employed. Leather tanneries are also unique environments for microbial growth. Leather tanneries are relatively open industrial systems which, as part of the tanning process, have a large influx of microorganisms from the process water, the hides or skins, as well as the other process materials. The incoming organisms vary with the incoming materials. Seasonal climate changes also affect the microorganisms in a tannery's process water.

A typical hide tanning process contains several stages to process the hide into an intermediate form, a tanned hide. In a typical chrome tanning process these stages include, for example, washing the hide with water and surfactant to remove debris; soaking the washed hide in an aqueous enzymatic bath (water, enzyme (protease), surfactant and alkali) to rehydrate the hide and remove interfibrillary proteins; dehairing the hide in a "hairbum" step using amines, sodium sulfide; and lime (CaO); deliming the hide by the addition of ammonium sulfate, water and multiple washes to remove alkali used during hair removal and adjust the pH of the hide; bating the hide (addition of a protease) to remove partially hydrolyzed collagen and keratin; pickling the hide (lowering the pH of the hide to prepare for the chrome tanning) by using an acid such as sulfuric acid; tanning by addition of chrome and basification (chrome fixation) by adding magnesium oxide; followed by rinsing. The resulting hide often has a blue color. At this stage the processed, or chrome-tanned, hide is known as a wet blue hide. The wet blue is only an intermediate which is normally stored or shipped to another location for further processing and finishing into leather.

Other methods of tanning, in addition to chrome tanning (which produces "wet blue" hides), which need protection from fungal contamination, include vegetable tanning, "wet white" tanned hides (including aluminum tanning, zirconium tanning, aldehyde tanning, and phosphonium tanning), and oil tanning. Examples of metal free tanning include use of oil, aldehyde or phosphonium salts. The tanned hides resulting from these tanning processes are known as vegetable-tanned hides, wet white hides and oil-tanned hides.

Each stage of a tanning process is a unique chemical environment as well as a unique microbiological environment. The microbial environment in a tanning process impacts the hide being processed. A hide does not typically need to be protected from fungal attack during the tanning process. After the tanning to yield a tanned hide the hide becomes subject to fungal attack and yields visible mold. It is also during the storage/shipping of tanned hides when fungal infestation occurs. A tanned hide is warm, moist, and a source of nutrients which promote fungal growth and mold which disfigures the appearance of the wet blue hide.

Biocides are used to protect tanned hides from fungi. Fungal attack discolors tanned hides, sometimes causing black spots. *Aspergillus niger* is a typical fungus that attacks leather products causing discoloration. This fungal discoloration is difficult to remove and can spoil tanned hides or the finished leather.

To protect the tanned hides (such as wet blue hides, vegetable-tanned hides, wet white hides, and oil-tanned hides), a fungicide is added to a tanning liquor used in the tanning process. Fungicides are often added during one of the late stages of the tanning process. Often a fungicide is added during the pickling stage which occurs under very acidic conditions. The fungicide must survive on the surface of the tanned hide to protect the hide from the time the tanning process is complete until the tanned hide is finished into true leather. To do this, a fungicide should itself survive in the chemical environment of the stage in which it is added as well as in the chemical environment of any subsequent stages. In order to be effective then, the fungicide should be stable under acid conditions, stable to UV-light, relatively unreactive with other tanning process chemicals, and have a high affinity for the hide being tanned. Other segments in the tanning process where fungicides could be employed include: prolonged shelf life of leather finish, fat liquor preservation and during the fatliquoring process.

A need exists in the leather industry for compounds that not only are effective fungicides but also survive the chemical environment of the hide tanning processes. This invention answers that need.

### SUMMARY OF THE INVENTION

The invention provides methods for controlling the fungal growth of at least one fungus on a tanned hide. This method comprises contacting a hide prior to, during, or after tanning with an effective amount of a cyanodithiocarbimate to control the growth of at least one fungus on the tanned hide. Cyanodithiocarbimates used are those of formula (I): 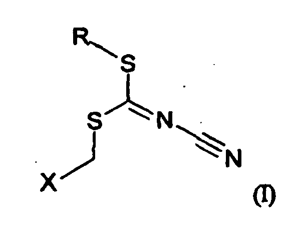 Cyanodithiocarbimates of formula (I) are useful in preventing the fungal attack, degradation, or deterioration of tanned hides, including but not limited to wet blue hides, wet white hides, vegetable-tanned hides, and oil-tanned hides, from the hide tanning process. New cyanodithiocarbimates of formula (I) represent a further embodiment of the invention.

### BRIEF DESCRIPTION OF THE DRAWINGS

Figure 1 depicts a general synthetic scheme for cyanodithiocarbimates of formula (I).

Figure 2 depicts the synthesis of hexyl chloromethyl cyanodithiocarbimate descried in Examples 1-3.

### DETAILED DESCRIPTION OF THE INVENTION

As discussed above, the invention relates to the use of cyanodithiocarbimates of formula (I) as fungicides in a tanning process to protect a tanned hide. 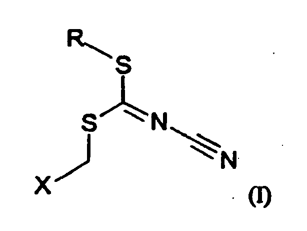 Mixtures of cyanodithiocarbimates of formula (I) may also be used.

In formula (I), X is a halogen, preferably Cl, Br, or I. Most preferably X is Cl.

The substituent R is a substituted or unsubstituted C₁-C₁₄ alkyl goup, a substituted or unsubstituted C₂-C₁₄ alkenyl group, a substituted or unsubstituted C₂-C₁₄ alkynyl group or a substituted or unsubstituted group defined by Y-Ar-(CH₂)ₘ- or by Z-(CH₂)ₙ-. In the Y-Ar-(CH₂)ₘ- group, m ranges from 0 to 3. Ar is a substituted or unsubstituted aryl group selected from phenyl and naphthyl. Y is H, halogen, NO₂, R¹, R¹O, or R¹R²N. When R is the group defined by Z-(CH₂)ₙ-, Z is NC, R¹O, R¹OC(O), or R¹OCH₂CH₂(OCH₂CH₂)ₚ where p ranges from 0 to 3. The value of n ranges from 0 to 3. R¹ and R² are independently H, substituted or unsubstituted C₁-C₅ alkyl.

In a preferred embodiment, R is a substituted or unsubstituted C₁-C₇ alkyl group, a substituted or unsubstituted C₂-C₇ alkenyl group, a substituted or unsubstituted group defined by Y-Ar-(CH₂)ₘ- or by Z-(CH₂)ₙ-. The value of n ranges from 0 to 3. In the Y-Ar-(CH₂)ₘ-group, m is 0 or I. Ar is preferably phenyl. Y is H, Cl, Br, I, NO₂, R¹, R¹O, or R¹R²N. When R is the group defined by Z-(CH₂)ₙ-, wherein Z is NC, R¹O, R¹OC(O), or R¹OCH₂CH₂(OCH₂CH₂)_{P} where p ranges from 0 to 3. R¹ and R² are independently H, methyl, or ethyl. Preferred cyanodithiocarbimates are shown below in Table 1.

For cyanodithiocarbimates of formula (I) the alkyl, alkenyl, or alkynyl groups may be branched or unbranched (*i.e*., a straight chain). An alkenyl or alkynyl group may be unsaturated at more than one position and may contain both carbon-carbon double bonds and carbon-carbon triple bonds. As indicated various groups may be substituted or unsubstituted by one or more groups that do not adversely effect the fungicidal activity of the cyanodithiocarbimate. Unsubstituted means the particular moiety carries hydrogen atoms on its constituent atoms, e.g. CH₃ for unsubstituted methyl. Substituted means that the group can carry typical functional groups know in organic chemistry. Such functional groups include, but are not limited to, halides, hydroxyl, thiols, amine groups, carboxylic acid groups; ketones, aldehydes, nitriles, nitro, azo, nitroso, ethers, thioethers and amides.

**Table 1: Preferred Cyanodithiocarbimates**

| X is Cl |
|---|
| R is -CH₃, -(CH₂)₂CH₃, -(CH₂)₃CH₃, -(CH₂)₅CH₃, -(CH₂)₇CH₃, -(CH₂)₁₁CH₃, - CH(CH₃)₂, -CH(CH₃)(CH₂)₃CH₃, -(CH₂)₂OH, -(CH₂)₃OH, -(CH₂CH₂O)₂CH₂CH₂OH -(CH₂)₂CO₂H, -CH₂CH₂CN, -CH₂CH=CH₂, CH₂C₆H₅ |

| X is Br |
|---|
| R is -(CH₂)₃CH₃, -CH₂C₆H₅ |

| X is I |
|---|
| R is -(CH₂)₃CH₃, -CH₂C₆H₅. |

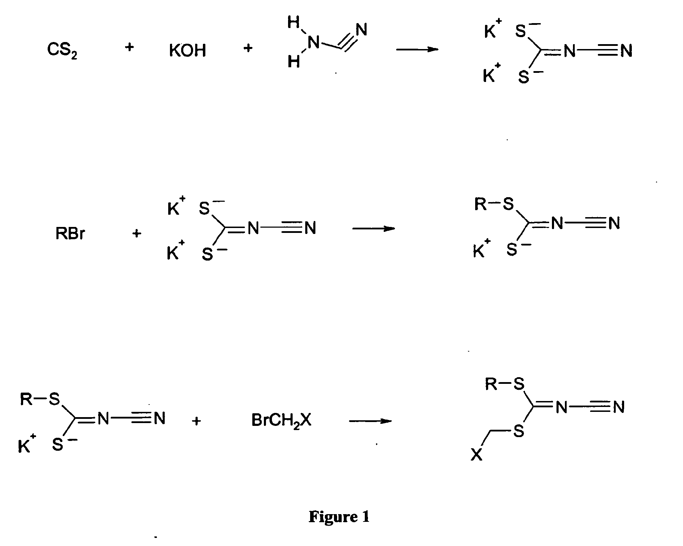

The cyanodithiocarbimates used in the methods of the invention may be prepared according to the general scheme shown in Fig. 1. To generate kabonate, cyanamide was reacted with carbon disulfide and KOH in ethanol under a blanket of nitrogen, with vigorous stirring. The temperature after addition was kept below 25°C. Sodium hydroxide could also have been used to generate the sodium salt, nabonate. Hexyl kabonate was produced by the slow addition of an acetone solution of hexyl bromide to a chilled aqueous solution of kabonate, containing a phase transfer catalyst. The mixture was allowed to come to room temperature and stirring was continued for several hours. The mixture was heated for several hours at 50°C. The acetone was removed under vacuum and the aqueous layer was extracted with methylene chloride or chloroform. The aqueous hexyl kabonate was added over a period of several hours to bromochloromethane (BCM) containing phase transfer catalyst. Heating was continued for six hours. After cooling, the aqueous layer was removed and the organic layer was washed with water, then dried over magnesium sulfate. The BCM was removed under vacuum to recover a pale orange liquid, hexyl chloromethyl cyanodithiocarbimate.

Starting materials to prepare other preferred cyanodithiocarbimates according to the general scheme shown in Fig. 1 include, for example, hexyl chloride, ethyl bromide, propyl bromide, isopropyl bromide, butyl bromide, hexyl bromide, 2-hexyl bromide, octyl bromide, dodecyl bromide, allyl bromide, methyl iodide, hexyl iodide, 3-bromo-1-propanol, chloroethanol, chloroethoxyethoxyethanol, bromopropionic acid, benzyl bromide, 4-methyl benzyl bromide, 4-Chlorobenzyl bromide, and bromopropionitrile. Published synthetic methods for cyanodithiocarbimates such as ethyl chloromethyl cyanodithiocarbimate, propyl chloromethyl cyanodithioicarbimate, isopropyl chloromethyl cyanodithiocarbimate, ally chloromethyl cyanodithiocarbimate, and benzyl chloromethyl cyanodithiocarbimate are reported in C. Fieseler, W. Walek and U. Thust, Tag.-Ber. Akad. Landwirtsch.-Wiss. DDR, Berlin (1990) 291. 317-321; and German Patent DD 275433 CYANOIMIDODITHIOCARBONATES AS WOOD PRESERVATIVES to W. Walek, J. Nauman, H. D. Pfeiffer, U. Thust, K. Trautner, C. Fieseler, M. Heschel, R. Hesse, H. Kirk and D. Mielke.

As discussed, the invention relates to a method for controlling the growth of at least one fungus on a tanned hide. The method comprises the step of contacting a hide prior to, during or after tanning with an effective amount of a cyanodithiocarbimate of formula (I):

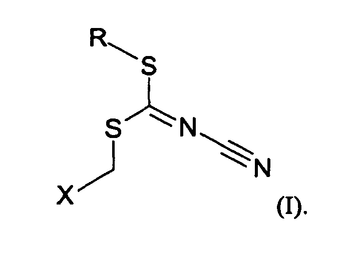

Controlling the growth of at least one fungus on a tanned hide means control to, at, or below a desired level and for a desired period of time for the particular substrate or system. This can vary from the complete prevention or inhibition of growth to control at a certain desired level and for a desired time. The use of a cyanodithiocarbimate as described here can, in many cases, reduce the total fungal count to undetectable limits and maintain the count at that level for a significant period of time. Preferably, the method is used to eliminate or prevent substantially all fungal growth on the tanned hide. Accordingly, the invention may be used to preserve a tanned hide.

According to the invention, a cyanodithiocarbimate is used to control fungal growth on a tanned hide after a tanning process. The hide may be any type of hide or skin that is tanned, for example cowhide, snake skin, alligator skin or sheep skin. The invention is particularly useful in controlling fungal growth on tanned hides during the time after tanning and until processing into finished leather goods. To achieve this control, during the tanning process the hide is contacted with an amount of a cyanodithiocarbimate effective to control the growth of at least one fungus on the tanned hide.

Types of tanning include mineral tanning (use of chromium, aluminum, zirconium, or titanium), vegetable tanning (use of natural extracts such as mimosa, quebracho, tara, or valonia), organic tannages (glutaraldehyde, THPS, or oxazolidine), and oil tanning (for example, use of fish oil to produce chamois). A number of stages comprise a typical tanning process, including, but not limited to, a pickling stage, a tannage addition stage, a basification stage (or fixation stage in the case of vegetable tanning), and a post-tan washing stage. Post-tanning may include a neutralization stage, a retanning stage, a dyeing stage, and a fatliquoring stage. A cyanodithiocarbimate of formula (I) may be used during all process stages in the tanning process in addition to those stages where aknown fungal growth may occur. In each stage, a cyanodithiocarbimate of formula (I) may be a component of the appropriate tanning liquor applied to the hide undergoing tanning. Preferably the hide is contacted with a cyanodithiocarbimate during the pickling stage.

Incorporating a cyanodithiocarbimate into a tanning liquor protects the hide from fungal deterioration subsequent to completion of the tanning process. Preferably, a cyanodithiocarbimate is uniformly dispersed, e.g. under agitation, into an appropriate liquor to be used in a tanning process. Typical tanning liquors include, for example, a mineral tanning liquor, a vegetable tanning liquor, an organic tanning liquor, an oil tanning liquor, a pickling liquor, a post-tan washing liquor, a retanning liquor, a dye liquor, and a fat liquor. This method of application ensures that the cyanodithiocarbimate applied to the hide protects against fungal attack, deterioration, or other microbiological degradation, particularly during storage and transporation of the tanned or pickled hide.

The concentration of the fungicide used to protect the tanned hides depends upon the process conditions used in the tanning process. According to the invention, a cyanodithiocarbimate of formula (I) may be used in similar amounts and manner similar to that used to apply other fungicides used in the tanning industry. The effective amount of the cyanodithiocarbimate necessary to achieve the desired degree of fungal control may vary somewhat depending on the particular hide to be protected, the conditions for fungal growth, and the degree of protection desired. The amount can be readily determined by one skilled in the art For a particular application, the amount of choice may be determined by routine testing of various amounts prior to treatment of the entire affected leather substrate. In general, an effective amount used on a substrate ranges from 0.0001 % to 4% (w/w); preferably 0.0001% to 0.2%. With aqueous systems, an effective amount may range from 5 to 1000 parts per million of the aqueous system, preferably from 10 to 500 parts per million, and more preferably be about 300 parts per million.

In addition to incorporating a cyanodithiocarbimate in a tanning liquor, fungal control may be accomplished by spraying the hide to be protected with a formulation of a cyanodithiocarbimate of formula (I). Fungal control may also be achieved by dipping or soaking the hide in a separate bath containing the cyanodithiocarbimate. Accordingly, the contacting step can also be accomplished by any means known in the art, spraying, soaking, dipping the hide. These can occur prior to tanning, between stages of the tanning process or after the tanning process is complete.

In the methods of the invention, the cyanodithiocarbimates of formula (I) may be formulated in various forms known in the art. For example, they may be prepared in liquid form as an aqueous solution, dispersion, emulsion, or suspension, a dispersion or suspension in a non-aqueous solvent, or as a solution by dissolving the compound(s) to be used in a solvent or combination of solvents. Suitable solvents include, but are not limited to, methyl ethers of glycols, M-pyrol or petroleum distillates. Diluents such as vegetable products including oils from: soybeans, pine trees, cottonseed, corn, canola, peanut, palm, rice, olive, tung nut, castor bean, linseed, citrus or datenut. The cyanodithiocarbimates may be prepared as a concentrate for dilution prior to its intended use. Common additives such as surfactants, emulsifiers, dispersants, and the like may be used as known in the art to increase solubility in a liquid composition or system, such as an aqueous composition or system. In many cases, the cyanodithiocarbimate compound(s) used may be solubilized by simple agitation.

Fungicidal compositions containing a cyanodithiocarbimate to be used in the method of the invention may also be formulated in solid form, for example as a powder or tablet, using means known in the art. In a preferred formulation for use, a liquid form of a cyanodithiocarbimate is deposited on a carrier such as diatomaceous earth, zeolites, kaolin, or a water-soluble salt matrix to form a powder or tablet.

In another embodiment, the invention relates to new cyanodithiocarbimates of formula (I): 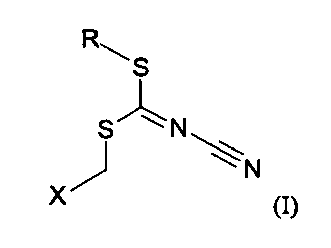 where:
X is a halogen;
R is a substituted or unsubstituted C₄-C₁₄ alkyl group, a substituted or unsubstituted C₂-C₁₄ alkenyl group with the proviso that it is not -CH₂CH=CH₂, a substituted or unsubstituted
C₂-C₁₄ alkynyl group, a substituted or unsubstituted group defined by Y-Ar-(CH₂)ₘ or by Z-(CH₂)ₙ-;
Ar is a substituted or unsubstituted aryl group selected from phenyl, and naphthyl;
Y is H, halogen, NO₂, R¹, R¹O, R¹R²N;
Z is NC, R¹O, R¹OC(O), R¹OCH₂CH₂(OCH₂CH₂)ₚ
m is 0, 2, or 3;
n ranges from 0 to 3;
p ranges from 0 to 3; and
R¹ and R² are independently H, substituted or unsubstituted C₁-C₅ alkyl.
Preferred cyanodithiocarbimates of formula (I) are those where:
R is a substituted or unsubstituted C₅-C₇ alkyl group, a substituted or unsubstituted C₂-C₇ alkenyl group with the proviso that it is not -CH₂CH=CH₂, a substituted or unsubstituted group defined by Y-Ar-(CH₂)ₘ₋ or by Z-(CH₂)ₙ-;
Ar is phenyl;
Y is H, Cl, Br, I, NO₂, R¹, R¹O, or R¹R²N;
Z is NC, R¹O, R¹OC(O), or R¹OCH₂CH₂(OCH₂CH₂)ₚ
m is 0; and
R¹ and R² are independently H, methyl, or ethyl.
More preferred cyanodithiocarbimates of formula (I) are those where:
X is Cl and R is -(CH₂)₅CH₃, -(CH₂)₇CH₃, -(CH₂)₁₁ CH₃ -CH(CH₃)₂,-CH(CH₃)(CH₂)₃CH₃, -(CH₂)₂OH, -(CH₂)₃OH, -(CH₂CH₂O)₂CH₂CH₂OH, -(CH₂)₂CO₂H,-CH₂CH₂CN, or -CH₂C₆H₅;
X is Br and R is -(CH₂)₃CH₃, or -CH₂C₆H₅; or
X is I and R is -(CH₂)₃CH₃, or CH₂C₆H₅.
A particularly preferred cyanodithiocarbimates of formula (I) is hexyl chloromethyl cyanodithiocarbimate.

### Examples

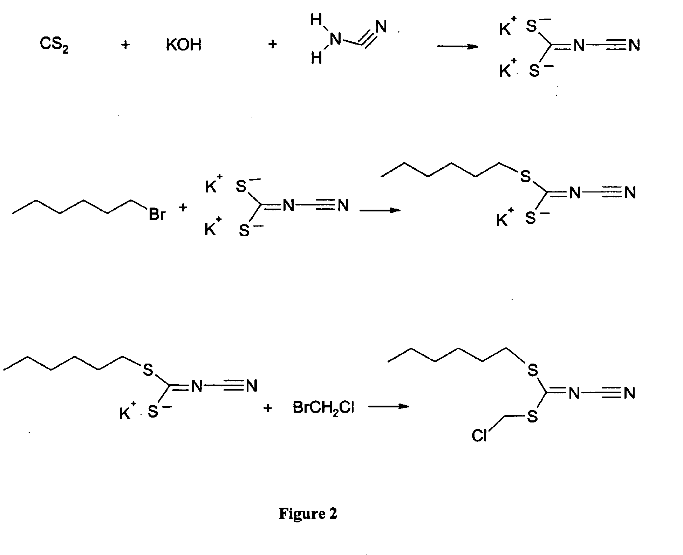

Examples 1- 3 describe the synthesis of hexyl chloromethyl cyanodithiocarbimate according to the reaction scheme shown in Fig. 2.

### Example 1 Kabonate^{™} (dipotassium cyanoclithiocarbimate)

Into 2 liters of ethanol was charged 500 grams of solid cyanamide. It was mixed until dissolved and filtered. It was then added to a 12 liter four neck flask, with mechanical stirrer, nitrogen blanket, addition funnel and thermometer and chill to 0°C with ice/water bath. To the cyanamide solution was added 790 ml carbon disulfide. In 8 liters of ethanol was dissolved 1547 grams of KOH (85%) and addition to the cyanamide/carbon disulfide was begun, keeping temperature below 25°C. After the addition was complete, mixing was continued overnight. The precipitate was collected by filtration and dried with a nitrogen flush.

### Example 2: Hexyl Kabonate^{™} (hexyl derivate of Kabonate^{™})

Into a 12 liter flask equipped with thermometer, condenser and mechanical stirrer, was added 364 grams of kabonate, 2.8 grams of tetrabutyl ammonium bromide (TBAB) and 2750 grams of water. Solution was mixed for ten minutes before slow addition of 5225 ml of acetone. The mixture was placed in a large ice bath and cooled to 10°C, before the addition of 310 mL bromohexane in acetone, through an addition funnel over a three hour period. The mixture was allowed to come to room temperature and vigorous stirring was continued overnight The ice bath was removed and heating mantle added. The mixture was heated at 50°C for one hour. The acetone was removed under vacuum. The aqueous layer was extracted with a small volume of chloroform or methylene chloride.

### Example 3: Hexyl chloromethyl cyanodithiocarbimate

To a 12 liter flask, 3200 mL of bromochloromethane (BCM) and 6 grams of tetrabutyl ammonium bromide (TBAB) was added. The mixture was heated to 50 °C. Aqueous hexyl kabonate was added over three hours through an addition funnel and heating was continued for six hours. After cooling, the solution was transferred to a separatory funnel. The aqueous layer was removed and the BCM layer was washed with water. The BCM layer was dried with magnesium sulfate and solids filtered. The BCM was removed under vacuum to recover pale orange liquid. Product was identified by NMR and IR. Purity was determined by HPLC to be 93%. The yield was greater than 90%.

### Example 4: Fungicidal Activity

The antifungal activities of compounds of formula (I) were measured by determining the inhibition of growth of *Aspergillus niger* in a standard liquid medium. The liquid growth medium is described as a solution of mineral salts + yeast extract (MSY). The MSY broth was prepared as described in ASTM G 21-70¹, and amended with glucose (10 g liter⁻¹) and yeast extract (1 g liter⁻¹). An aliquot (250 µL) of sterile medium was dispensed into each test well of a standard 96-well microplate (Coming No. 430247). Stock solutions of test compounds were prepared by dissolving the materials in 9:1 (v/v) solution of acetone:methanol. Appropriate volumes of stock solutions were added to the test wells in order to achieve the desired ppm levels. Each test well (plus controls) was then inoculated with 5 µL of a standardized suspension *of Aspergillus niger.* The cell suspension was prepared by suspending viable cells from a slant of potato dextrose agar into sterile, deionized water. The suspension was then adjusted to provide OD₆₈₆ = 0.28. This density contains approximately 2.5 x 10⁷ CFU mL⁻¹ (CFU = Colony Forming Units). The microplates were incubated in the dark for four days at 28°C. The optical density for each well at 686 nm was then recorded automatically using a SpectraMax 340 microplate reader (Molecular Devices Corp., Sunnyvale, CA). All wells were visually inspected in order to corroborate data from the instrument. Test wells with an OD ≤ 0.05 were judged to exhibit complete inhibition of cellular growth. The results are shown in Table 2.
¹ "Standard Practice for Determining Resistance of Synthetic Polymeric Materials to Fungi",ASTM Standards on Materials and Environmental Microbiology, 1^{st}. Ed., 1987.

**Table 2**

| Compounds of Formula (I) | | |
|---|---|---|
| **X** | **R** | **Antifungal MIC** |
| Cl | -CH₂CHC=CH₂ | 1.0 |
| | -(CH₂)₇CH₃ | 2.0 |
| | -CH₃ | 1.0 |
| | -(CH₂)ₙCH₃ | 6.0 |
| | -(CH₂)₅CH₃ | 0.2 |
| | -(CH₂)₃CH₃ | 0.4 |
| | -(CH₂)₂CH₃ | 0.5 |
| | -CH₂C₆H₅ | 10 |
| | -CH₂(CH₃)₂ | 0.5 |
| | -(CH₂)₃OH | 6.0 |
| | -(CH₂CH₂O)₂CH₂CH₂OH | 6.0 |
| | -(CH₂)₂OH | 6.0 |
| | -(CH₂)₂CO₂H | > 100 |
| Br | -(CH₂)₃CH₃ | 1.4 |
| | -CH₂C₆H₅ | 2.0 |
| I | -(CH₂)₃CH₃ | 2.0 |
| | -CH₂C₆H₅ | 5.0 |

### Example 5: Performance Testing of the Hexyl chloromethyl cyanodithiocarbimate in a Tannery

A methyl carbitol-based formulation containing 30% w/w of hexyl chloromethyl cyanodithiocarbimate was tested in a commercial tannery. Eighty-two cowhides were subjected to a standard production run using chrome tanning. The test formulation was added to the float (tanning liquid) at a concentration of 0.1 % by weight providing a concentration of 300 ppm of active ingredient hexyl chloromethyl cyanodithiocarbimate. At the completion of the tanning process, five tanned hides ("wet blues") were chosen randomly. Numerous test pieces (12 x 14 cm) were cut from the butt, belly, and neck regions of each of the five hides. A total of 47 test pieces of wet blue obtained were coded relative to hide source and position, and each piece was suspended in a tropical chamber. The tropical chamber was a mold-infested humidity chamber designed to severely challenge the test fungicide applied during the tanning process. Each test piece was visually examined on a weekly basis for four weeks for the presence or absence and extent of possible mold growth. After four weeks of incubation in the tropical chamber, each piece tanned in the presence of hexyl chloromethyl cyanodithiocarbimate was free of fungal growth (mold). This is the type of performance desired of a fungicide by the hide tanning industry.

## Claims

1. A method for controlling the growth of at least one fungus on a tanned animal hide comprising the step of contacting a hide prior to, during or after tanning with a cyanodithiocarbimate of formula (I): 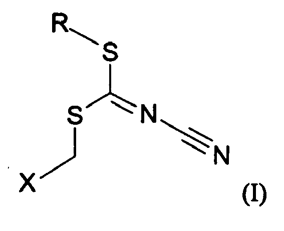
X is a halogen;
R is a substituted or unsubstituted C₁-C₁₄ alkyl group, a substituted or unsubstituted C₂-C₁₄ alkenyl group, a substituted or unsubstituted C₂-C₁₄ alkynyl group, a substituted or unsubstituted group defined by Y-Ar-(CH₂)ₘ- or by Z-(CH₂)ₙ-;
Ar is a substituted or unsubstituted aryl group selected from phenyl, and naphthyl;
Y is H, halogen, NO₂, R¹, R¹O, or R¹R²N;
Z is NC, R¹O, R¹OC(O), or R¹OCH₂CH₂(OCH₂CH₂)ₚ
m ranges from 0 to 3;
n ranges from 0 to 3;
p ranges from 0 to 3; and
R¹ and R² are independently H, substituted or unsubstituted C₁-C₅ alkyl; in an amount effective to control the growth of at least one fungus on the tanned hide.

2. A method of claim 1, wherein:
X is Cl, Br, or I;
R is a substituted or unsubstituted C₁-C₇ alkyl group, a substituted or unsubstituted C₂-C₇ alkenyl group, a substituted or unsubstituted group defined by Y-Ar-(CH₂)ₘ or by Z-(CH₂)ₙ;
Ar is phenyl;
Y is H, Cl, Br, I, NO₂, R¹, R¹O, or R¹R²N;
Z is NC, R¹O, R¹OC(O), or R¹OCH₂CH₂(OCH₂CH₂)ₚ
m is 0 or 1; and
R¹ and R² are independently H, methyl, or ethyl.

3. A method of claim 1, wherein
X is Cl and R is -CH₃, -(CH₂)₂CH₃, -(CH₂)₃CH₃, -(CH₂)₅CH₃, -(CH₂)₇CH₃,-(CH₂)₁₁CH₃, -CH(CH₃)₂, -CH(CH₃)(CH₂)₃CH₃, -(CH₂)₂OH, -(CH₂)₃OH, - (CH₂CH₂O)₂CH₂CH₂OH, -(CH₂)₂CO₂H, -CH₂CH₂CN, -CH₂CH=CH₂, or CH₂C₆H₅;
X is Br and R is -(CH₂)₃CH₃, or -CH₂C₆H₅; or
X is I and R is -(CH₂)₃CH₃, or -CH₂C₆H₅.

4. A method of claim 1, wherein the cyanodithiocarbimate of formula (I) is hexyl chloromethyl cyanodithiocarbimate.

5. A method of claim 1, wherein the leather substrate is a wet blue hide a wet white hide, a vegetable-tanned hide or an oil-tanned hide.

6. A method of claim 3, wherein the leather substrate is a wet blue hide a wet white hide, a vegetable-tanned hide or an oil-tanned hide.

7. A method of claim 4, wherein the leather substrate is a wet blue hide a wet white hide, a vegetable-tanned hide or an oil-tanned hide.

8. A method for controlling the growth of microorganisms on a hide during a leather tanning process comprising the step of contacting a hide susceptible to fungal growth with a tanning liquor containing a cyanodithiocarbimate of formula (I): 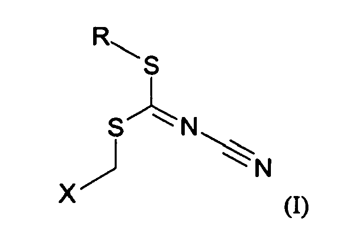
X is a halogen
R is a substituted or unsubstituted C₁-C₁₄ alkyl group, a substituted or unsubstituted C₂-C₁₄ alkenyl group, a substituted or unsubstituted C₂-C₁₄ alkynyl group, a substituted or unsubstituted group defined by Y-Ar-(CH₂)ₘ- or by Z-(CH₂)ₙ-;
Ar is a substituted or unsubstituted aryl group selected from phenyl, and naphthyl;
Y is H, halogen, NO₂, R¹, R¹O, or R¹R²N;
Z is NC, R¹O, R¹OC(O), or R¹OCH₂CH₂(OCH₂CH₂)
m ranges from 0 to 3;
n ranges from 0 to 3;
p ranges from 0 to 3; and
R¹ and R² are independently H, substituted or unsubstituted C₁-C₅ alkyl; in an amount effective to control the growth of at least one fungus on the hide.

9. A method of claim 8, wherein
R is a substituted or unsubstituted C₁-C₇ alkyl group, a substituted or unsubstituted C₂-C₇ alkenyl group, a substituted or unsubstituted group defined by Y-Ar-(CH₂)ₘ- or by Z-(CH₂)ₙ-;
Ar is phenyl;
Y is H, Cl, Br, I, NO₂, R¹, R¹O, or R¹R²N;
Z is NC, R¹O, R¹OC(O), or R¹OCH₂CH₂(OCH₂CH₂)ₚ
m is 0 or 1; and
R¹ and R² are independently H, methyl, or ethyl.

10. A method of claim 8, wherein
X is Cl and R is -CH₃, -(CH₂)₂CH₃, -(CH₂)₃CH₃, -(CH₂)₅CH₃, -(CH₂)₇CH₃, - (CH₂)ₙCH₃, -CH(CH₃)₂, -CH(CH₃)(CH₂)₃CH₃, -(CH₂)₂OH, -(CH₂)₃OH, - (CH₂CH₂O)₂CH₂CH₂OH, -(CH₂)₂CO₂H, -CH₂CH₂CN, -CH₂CH=CH₂, or -CH₂C₆H₅;
X is Br and R is -(CH₂)₃CH₃, or -CH₂C₆H₅; or
X is I and R is -(CH₂)₃CH₃, or -CH₂C₆H₅.

11. A method of claim 8, wherein the cyanodithiocarbimate of formula (I) is hexyl chloromethyl cyanodithiocarbimate.

12. A method of claim 8, wherein the hide is a wet blue hide, a wet white hide, a vegetable-tanned hide or an oil-tanned hide.

13. A method of claim 10, wherein the hide is a wet blue hide, a wet white hide, a vegetable-tanned hide or an oil-tanned hide.

14. A method of claim 11 , wherein the hide is a wet blue hide, a wet white hide, a vegetable-tanned hide or an oil-tanned hide.

15. A method of claim 8, wherein the tanning liquor is a pickling liquor.

16. A method of claim 8, wherein the cyanodithiocarbimate of formula (I) is present in the tanning liquor in an amount ranging from 5 to 500 parts per million.

17. A liquor for use in a leather-tanning process comprising a cyanodithiocarbimate of formula (I): 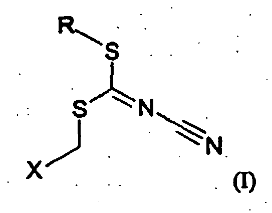
X is a halogen;
R is a substituted or unsubstituted C₁-C₁₄ alkyl group, a substituted or unsubstituted C₂-C₁₄ alkenyl group, a substituted or unsubstituted C₂-C₁₄ alkynyl group, a substituted or unsubstituted group defined by Y-Ar-(CH₂)ₘ₋ or by Z-(CH₂)ₙ-;
Ar is a substituted or unsubstituted aryl group selected from phenyl, and naphthyl;
Y is H, halogen, NO₂, R¹, R¹O, or R¹R²N;
Z is NC, R¹O, R¹OC(O), or R¹OCH₂CH₂(OCH₂CH₂)_{P};
in ranges from 0 to 3;
n ranges from 0 to 3;
p ranges from 0 to 3; and
R¹ and R² are independently H, substituted or unsubstituted C₁-C₅ alkyl;
wherein the liquor is selected from a pickling liquor, a chrome-tanning liquor, a white-tanning liquor, a vegetable-tanning liquor, an oil-tanning liquor, a post-tan washing liquor, a retanning liquor, a dye liquor, and a fat liquor; and wherein the cyanodithiocarbimate of formula (I) is present in the tanning liquor in an amount ranging from 5 to 500 parts per million.

18. A liquor of claim 17, wherein
R is a substituted or unsubstituted C₁-C₇ alkyl group, a substituted or unsubstituted C₂-C₇ alkenyl group, a substituted or unsubstituted group defined by Y-Ar-(CH₂)ₘ- or by Z- (CH₂)ₙ;
Ar is phenyl;
Y is H, Cl, Br, I, NO₂, R¹, R¹O, or R¹R²N;
Z is NC, R¹O, R¹OC(O), or R¹OCH₂CH₂(OCH₂CH₂)_{P};
m is 0 or 1 ; and
R¹ and R² are independently H, methyl, or ethyl.

19. A liquor of claim 17, wherein
X is Cl and R is -CH₃, -(CH₂)₂CH₃, -(CH₂)₃CH₃, -(CH₂)₅CH₃, -(CH₂)₇CH₃, (CH₂)₁₁ CH₃, -CH(CH₃)₂, -CH(CH₃)(CH₂)₃CH₃, -(CH₂)₂OH, -(CH₂)₃OH, -(CH₂CH₂O)₂CH₂CH₂OH, -(CH₂)₂CO₂H, -CH₂CH₂CN, -CH₂CH=CH₂, or -CH₂C₆H₅;
X is Br and R is -(CH₂)₃CH₃, or -CH₂C₆H₅; or
X is I and R is -(CH₂)₃CH₃, or CH₂C₆H₅.

20. A liquor of claim 17, wherein the cyanodithiocarbimate of formula (I) is hexyl chloromethyl cyanodithiocarbimate.

21. A liquor of claim 17 , wherein the liquor is a pickling liquor.

22. A liquor of claim 17, further comprising a solvent selected from methyl ethers of glycols, M-pyrol, and petroleum distillates; and a diluent selected from soybean oil, pine tree oil, cottonseed oil, corn oil, canola oil, peanut oil, palm oil, rice oil, olive oil, tung nut oil, castor bean oil, linseed oil, citrus oil, and date nut oil.

23. A cyanodithiocarbimate of formula (I): 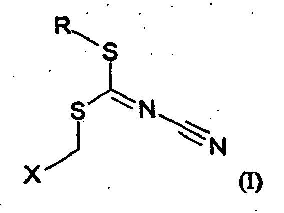
X is a halogen;
R is a substituted or unsubstituted C₄-C₁₄alkyl group, a substituted or unsubstituted C₂-C₁₄ alkenyl group with the proviso that it is not -CH₂CH=CH₂, a substituted or unsubstituted C₂-C₁₄ alkynyl group, a substituted or unsubstituted group defined by Y-Ar-(CH₂)ₘ- or by Z- (CH₂)ₙ-;
Ar is a substituted or unsubstituted aryl group selected from phenyl, and naphthyl;
Y is H, halogen, NO₂, R¹, R¹O,R¹R²N;
Z is NC, R¹O, R¹OC(O), R¹OCH₂CH₂(OCH₂CH₂)ₚ;
m is 0, 2, or 3;
n ranges from 0 to 3;
p ranges from 0 to 3; and
R¹ and R² are independently H, substituted or unsubstituted C₁-C₅ alkyl.

24. At cyanodithiocarbimate of claim 23, wherein
R is a substituted or unsubstituted C₅-C₇ alkyl group, a substituted or unsubstituted C₂-C₇ alkenyl group with the proviso that it is not -CH₂CH=CH₂, a substituted or unsubstituted group defined by Y-Ar-(CH₂)ₘ- or by Z-(CH₂)ₙ-;
Ar is phenyl;
Y is H, Cl, Br, I, NO₂, R¹, R¹O, or R¹R²N;
Z is NC,R¹O, R¹OC(O), or R¹OCH₂CH₂(OCH₂CH₂)ₚ;
m is 0; and
R¹ and R² are independently H, methyl, or ethyl.

25. A cyanodithiocarbimate of claim 23, wherein
X is Cl and R is -(CH₂)₅CH₃, -(CH₂)₇CH₃, -(CH₂)₁₁CH₃, -(CH₂)CH(CH₃)₂, -CH(CH₃)(CH₂)₃CH₃, -(CH₂)₂OH, -(CH₂)₃OH, -(CH₂CH₂O)₂CH₂CH₂OH, -(CH₂)₂CO₂H, -CH₂CH₂CN, -CH₂C₆H₅;
X is Br and R is -(CH₂)₃CH₃, or -CH₂C₆H₅; or
X is I and R is -(CH₂)₃CH₃, or CH₂C₆H₅.

26. Hexyl chloromethyl cyanodithiocarbimate.

## Patentansprüche

1. Verfahren zum Bekämpfen des Wachstums von wenigstens einem Pilz auf einer gegerbten Tierhaut, umfassend den Schritt des Inkontaktbringens einer Haut vor, während oder nach dem Gerben mit einem Cyanodithiocarbimat der Formel (I): 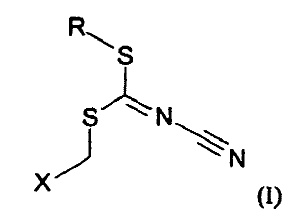
X ist ein Halogen;
R ist eine substituierte oder unsubstituierte C₁-C₁₄-Alkylgruppe, eine substituierte oder unsubstituierte C₂-C₁₄-Alkenylgruppe, eine substituierte oder unsubstituierte C₂-C₁₄-Alkinylgruppe, eine substituierte oder unsubstituierte Gruppe, die durch Y-Ar-(CH₂)ₘ- oder durch Z-(CH₂)ₙ- definiert ist;
Ar ist eine substituierte oder unsubstituierte Arylgruppe, ausgewählt aus Phenyl und Naphthyl;
Y ist H, Halogen, NO₂, R¹, R¹O oder R¹R²N;
Z ist NC, R¹O, R¹OC(O) oder R¹OCH₂CH₂(OCH₂CH₂)_{P}
m liegt im Bereich von 0 bis 3;
n liegt im Bereich von 0 bis 3;
p liegt im Bereich von 0 bis 3; und
R¹ und R² sind unabhängig voneinander H, substituiertes oder unsubstituiertes C₁-C₅-Alkyl;
in einer Menge, die zum Bekämpfen des Wachstums von wenigstens einem Pilz auf der gegerbten Haut wirksam ist.

2. Verfahren nach Anspruch 1, wobei:
X ist CI, Br oder I;
R ist eine substituierte oder unsubstituierte C₁-C₇-Alkylgruppe, eine substituierte oder unsubstituierte C₂-C₇-Alkenylgruppe, eine substituierte oder unsubstituierte Gruppe, die durch Y-Ar-(CH₂)ₘ- oder Z-(CH₂)ₙ- definiert ist;
Ar ist Phenyl;
Y ist H, Cl, Br, I, NO₂, R¹, R¹O oder R¹R²N;
Z ist NC, R¹O, R¹OC(O) oder R¹OCH₂CH₂(OCH₂CH₂)ₚ
m ist 0 oder 1; und
R¹ und R² sind unabhängig voneinander H, Methyl oder Ethyl.

3. Verfahren nach Anspruch 1, wobei
X ist Cl und R ist -CH₃, -(CH₂)₂CH₃, -(CH₂)₃CH₃, -(CH₂)₅CH₃, -(CH₂)₇CH₃, -(CH₂)₁₁CH₃, -CH(CH₃)₂, -CH(CH₃)(CH₂)₃CH₃, -(CH₂)₂OH, -(CH₂)₃OH, -(CH₂CH₂O)₂CH₂CH₂OH, -(CH₂)₂CO₂H, -CH₂CH₂CN, -CH₂CH=CH₂ oder CH₂C₆H₅;
X ist Br und R ist -(CH₂)₃CH₃ oder -CH₂C₆H₅; oder
X ist I und R ist -(CH₂)₃CH₃ oder -CH₂C₆H₅.

4. Verfahren nach Anspruch 1, wobei das Cyanodithiocarbimat der Formel (I) Hexylchlormethylcyanodithiocarbimat ist.

5. Verfahren nach Anspruch 1, wobei das Ledersubstrat eine feuchte chromgegerbte Haut (Wet Blue-Haut), eine feuchte synthetisch gegerbte Haut (Wet White-Haut), eine pflanzlich gegerbte Haut oder eine ölgegerbte (sämischgare) Haut ist.

6. Verfahren nach Anspruch 3, wobei das Ledersubstrat eine feuchte chromgegerbte Haut (Wet Blue-Haut), eine feuchte synthetisch gegerbte Haut (Wet White-Haut), eine pflanzlich gegerbte Haut oder eine ölgegerbte (sämischgare) Haut ist.

7. Verfahren nach Anspruch 4, wobei das Ledersubstrat eine feuchte chromgegerbte Haut (Wet Blue-Haut), eine feuchte synthetisch gegerbte Haut (Wet White-Haut), eine pflanzlich gegerbte Haut oder eine ölgegerbte (sämischgare) Haut ist.

8. Verfahren zum Bekämpfen des Wachstums von Mikroorganismen auf einer Haut während eines Ledergerbverfahrens, umfassend den Schritt des Inkontaktbringens einer für Pilzwachstum anfälligen Haut mit einer Gerbbrühe, die ein Cyanodithiocarbimat der Formel (I) enthält: 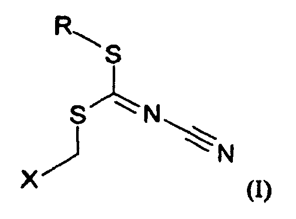
X ist ein Halogen
R ist eine substituierte oder unsubstituierte C₁-C₁₄-Alkylgruppe, eine substituierte oder unsubstituierte C₂-C₁₄-Alkenylgruppe, eine substituierte oder unsubstituierte C₂-C₁₄-Alkinylgruppe, eine substituierte oder unsubstituierte Gruppe, die durch Y-Ar-(CH₂)ₘ- oder durch Z-(CH₂)ₙ- definiert ist;
Ar ist eine substituierte oder unsubstituierte Arylgruppe, ausgewählt aus Phenyl und Naphthyl;
Y ist H, Halogen, NO₂, R¹, R¹O oder R¹R²N;
Z ist NC, R¹O, R¹OC(O) oder R¹OCH₂CH₂(OCH₂CH₂)ₚ
m liegt im Bereich von 0 bis 3;
n liegt im Bereich von 0 bis 3;
p liegt im Bereich von 0 bis 3; und
R¹ und R² sind unabhängig voneinander H, substituiertes oder unsubstituiertes C₁-C₅-Alkyl;
in einer Menge, die zum Bekämpfen des Wachstums von wenigstens einem Pilz auf der Haut wirksam ist.

9. Verfahren nach Anspruch 8, wobei
R ist eine substituierte oder unsubstituierte C₁-C₇-Alkylgruppe, eine substituierte oder unsubstituierte C₂-C₇-Alkenylgruppe, eine substituierte oder unsubstituierte Gruppe, die durch Y-Ar-(CH₂)ₘ- oder durch Z-(CH₂)ₙ- definiert ist;
Ar ist Phenyl;
Y ist H, Cl, Br, I, NO₂, R¹, R¹O oder R¹R²N;
Z ist NC, R¹O, R¹OC(O) oder R¹OCH₂CH₂(OCH₂CH₂)ₚ
m ist 0 oder 1; und
R¹ und R² sind unabhängig voneinander H, Methyl oder Ethyl.

10. Verfahren nach Anspruch 8, wobei
X ist Cl und R ist -CH₃, -(CH₂)₂CH₃, -(CH₂)₃CH₃, -(CH₂)₅CH₃, -(CH₂)₇CH₃, -(CH₂)ₙCH₃, -CH(CH₃)₂, -CH(CH₃)(CH₂)₃CH₃, -(CH₂)₂OH, -(CH₂)₃OH, -(CH₂CH₂O)₂CH₂CH₂OH, -(CH₂)₂CO₂H, -CH₂CH₂CN, -CH₂CH=CH₂ oder -CH₂C₆H₅;
X ist Br und R ist -(CH₂)₃CH₃ oder -CH₂C₆H₅; oder
X ist I und R ist -(CH₂)₃CH₃ oder -CH₂C₆H₅.

11. Verfahren nach Anspruch 8, wobei das Cyanodithiocarbimat der Formel (I) Hexylchlormethylcyanodithiocarbimat ist.

12. Verfahren nach Anspruch 8, wobei die Haut eine feuchte chromgegerbte Haut (Wet Blue-Haut), eine feuchte synthetisch gegerbte Haut (Wet White-Haut), eine pflanzlich gegerbte Haut oder eine ölgegerbte (sämischgare) Haut ist.

13. Verfahren nach Anspruch 10, wobei die Haut eine feuchte chromgegerbte Haut (Wet Blue-Haut), eine feuchte synthetisch gegerbte Haut (Wet White-Haut), eine pflanzlich gegerbte Haut oder eine ölgegerbte (sämischgare) Haut ist.

14. Verfahren nach Anspruch 11, wobei die Haut eine feuchte chromgegerbte Haut (Wet Blue-Haut), eine feuchte synthetisch gegerbte Haut (Wet White-Haut), eine pflanzlich gegerbte Haut oder eine ölgegerbte (sämischgare) Haut ist.

15. Verfahren nach Anspruch 8, wobei die Gerbbrühe eine Pickelbrühe ist.

16. Verfahren nach Anspruch 8, wobei das Cyanodithiocarbimat der Formel (I) in der Gerbbrühe in einer Menge im Bereich von 5 bis 500 Teilen pro Million vorhanden ist.

17. Brühe zur Verwendung in einem Ledergerbverfahren, umfassend ein Cyanodithiocarbimat der Formel (I): 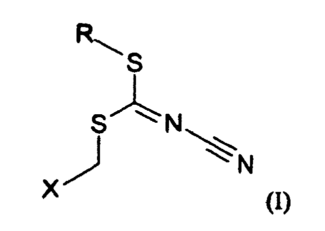
X ist ein Halogen;
R ist eine substituierte oder unsubstituierte C₁-C₁₄-Alkylgruppe, eine substituierte oder unsubstituierte C₂-C₁₄-Alkenylgruppe, eine substituierte oder unsubstituierte C₂-C₁₄-Alkinylgruppe, eine substituierte oder unsubstituierte Gruppe, die durch Y-Ar-(CH₂)ₘ- oder durch Z-(CH₂)ₙ- definiert ist;
Ar ist eine substituierte oder unsubstituierte Arylgruppe, ausgewählt aus Phenyl und Naphthyl;
Y ist H, Halogen, NO₂, R¹, R¹O oder R¹R²N;
Z ist NC, R¹O, R¹OC(O) oder R¹OCH₂CH₂(OCH₂CH₂)ₚ;
m liegt im Bereich von 0 bis 3;
n liegt im Bereich von 0 bis 3;
p liegt im Bereich von 0 bis 3; und
R¹ und R² sind unabhängig voneinander H, substituiertes oder unsubstituiertes C₁-C₅-Alkyl;
wobei die Brühe ausgewählt ist aus einer Pickelbrühe, einer Chromgerbbrühe, einer Weißgerbbrühe, einer pflanzlichen Gerbbrühe, einer Ölgerbbrühe bzw. Sämischgerbbrühe (oil-tanning liquor), einer nach dem Gerben anzuwendenden Waschflüssigkeit (post-tan washing liquor), einer Nachgerbbrühe, einer Farbbrühe und einer Fettbrühe; und wobei das Cyanodithiocarbimat der Formel (I) in der Gerbbrühe in einer Menge im Bereich von 5 bis 500 Teilen pro Million vorhanden ist.

18. Brühe nach Anspruch 17, wobei
R ist eine substituierte oder unsubstituierte C₁-C₇-Alkylgruppe, eine substituierte oder unsubstituierte C₂-C₇-Alkenylgruppe, eine substituierte oder unsubstituierte Gruppe, die durch Y-Ar-(CH₂)ₘ- oder durch Z-(CH₂)ₙ- definiert ist;
Ar ist Phenyl;
Y ist H, Cl, Br, I, NO₂, R¹, R¹O oder R¹R²N;
Z ist NC, R¹O, R¹OC(O) oder R¹OCH₂CH₂(OCH₂CH₂)ₚ;
m ist 0 oder 1; und
R¹ und R² sind unabhängig voneinander H, Methyl oder Ethyl.

19. Brühe nach Anspruch 17, wobei
X ist Cl und R ist -CH₃, -(CH₂)₂CH₃, -(CH₂)₃CH₃, -(CH₂)₅CH₃, -(CH₂)₇CH₃, -(CH₂)₁₁CH₃, -CH(CH₃)₂, -CH(CH₃)(CH₂)₃CH₃, -(CH₂)₂OH, -(CH₂)₃OH, -(CH₂CH₂O)₂CH₂CH₂OH, -(CH₂)₂CO₂H, -CH₂CH₂CN, -CH₂CH=CH₂ oder -CH₂C₆H₅;
X ist Br und R ist -(CH₂)₃CH₃ oder -CH₂C₆H₅; oder
X ist I und R ist -(CH₂)₃CH₃ oder -CH₂C₆H₅.

20. Brühe nach Anspruch 17, wobei das Cyanodithiocarbimat der Formel (I) Hexylchlormethylcyanodithiocarbimat ist.

21. Brühe nach Anspruch 17, wobei die Brühe eine Pickelbrühe ist.

22. Brühe nach Anspruch 17, außerdem umfassend ein Lösungsmittel, ausgewählt aus Methylethern von Glycolen, M-Pyrol und Petroleumdestillaten; und ein Verdünnungsmittel, ausgewählt aus Sojabohnenöl, Kiefernöl, Baumwollsamenöl, Maisöl, Canolaöl, Erdnussöl, Palmöl, Reisöl, Olivenöl, Tungöl, Riziniusöl, Leinsamenöl, Citrusöl und Dattelöl.

23. Cyanodithiocarbimat der Formel (I): 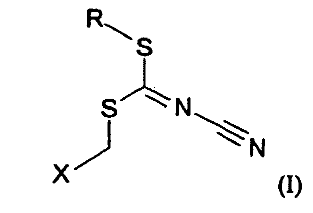
X ist ein Halogen;
R ist eine substituierte oder unsubstituierte C₄-C₁₄-Alkylgruppe, eine substituierte oder unsubstituierte C₂-C₁₄-Alkenylgruppe mit der Maßgabe, dass sie nicht -CH₂CH=CH₂ ist, eine substituierte oder unsubstituierte C₂-C₁₄-Alkinylgruppe, eine substituierte oder unsubstituierte Gruppe, die durch Y-Ar-(CH₂)ₘ- oder durch Z-(CH₂)ₙ- definiert ist;
Ar ist eine substituierte oder unsubstituierte Arylgruppe, ausgewählt aus Phenyl und Naphthyl;
Y ist H, Halogen, NO₂, R¹, R¹O, R¹R²N;
Z ist NC, R¹O, R¹OC(O), R¹OCH₂CH₂(OCH₂CH₂)ₚ;
m ist 0, 2 oder 3;
n liegt im Bereich von 0 bis 3;
p liegt im Bereich von 0 bis 3; und
R¹ und R² sind unabhängig voneinander H, substituiertes oder unsubstituiertes C₁-C₅-Alkyl.

24. Cyanodithiocarbimat nach Anspruch 23, wobei
R ist eine substituierte oder unsubstituierte C₅-C₇-Alkylgruppe, eine substituierte oder unsubstituierte C₂-C₇-Alkenylgruppe, mit der Maßgabe, dass sie nicht -CH₂CH=CH₂ ist, eine substituierte oder unsubstituierte Gruppe, die durch Y-Ar-(CH₂)ₘ- oder durch Z-(CH₂)ₙ- definiert ist;
Ar ist Phenyl;
Y ist H, Cl, Br, I, NO₂, R¹, R¹O oder R¹R²N;
Z ist NC, R¹O, R¹OC(O) oder R¹OCH₂CH₂(OCH₂CH₂)ₚ;
m ist 0; und
R¹ und R² sind unabhängig voneinander H, Methyl oder Ethyl.

25. Cyanodithiocarbimat nach Anspruch 23, wobei
X ist Cl und R ist -(CH₂)₅CH₃, -(CH₂)₇CH₃, -(CH₂)₁₁CH₃, -(CH₂)CH(CH₃)₂, -CH(CH₃)(CH₂)₃CH₃, -(CH₂)₂OH, -(CH₂)₃OH, -(CH₂CH₂O)₂CH₂CH₂OH, -(CH₂)₂CO₂H, -CH₂CH₂CN, -CH₂C₆H₅;
X ist Br und R ist -(CH₂)₃CH₃ oder -CH₂C₆H₅; oder
X ist I und R ist -(CH₂)₃CH₃ oder -CH₂C₆H₅.

26. Hexylchlormethylcyanodithiocarbimat.

## Revendications

1. Procédé de contrôle de la croissance d'au moins un champignon sur une peau animale tannée comprenant l'étape consistant à mettre en contact une peau avant, pendant ou après le tannage avec un cyanodithiocarbamate de formule (I) : 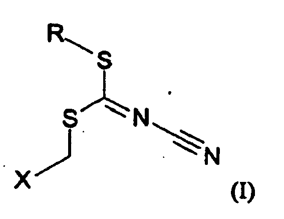
X est un halogène ;
R est un groupe alkyle en C₁-C₁₄ substitué ou non substitué, un groupe alcényle en C₂-C₁₄ substitué ou non substitué, un groupe alcynyle en C₂-C₁₄ substitué ou non substitué, un groupe substitué ou non substitué défini par Y-Ar-(CH₂)ₘ- ou par Z- (CH₂)ₙ- ;
Ar est un groupe aryle substitué ou non substitué choisi parmi le phényle et le naphthyle ;
Y est un H, un halogène, un NO₂, R¹, R¹O ou R¹R²N ;
Z est un NC, R¹O, R¹OC (O) ou R¹OCH₂CH₂ (OCH₂CH₂)ₚ ;
m est compris entre 0 et 3 ;
n est compris entre 0 et 3 ;
p est compris entre 0 et 3 ; et
R¹ et R² sont indépendamment un H, un alkyle en C₁-C₅ substitué ou non substitué ;
dans une quantité efficace pour contrôler la croissance d'au moins un champignon sur la peau tannée.

2. Procédé selon la revendication 1, dans lequel :
X est un Cl, Br ou I ;
R est un groupe alkyle en C₁-C₇ substitué ou non substitué, un groupe alcényle en C₂-C₇ substitué ou non substitué, un groupe substitué ou non substitué défini par Y-Ar-(CH₂)ₘ- ou par Z-(CH₂)ₙ- ;
Ar est un phényle ;
Y est un H, Cl, Br, I, NO₂, R¹, R¹O ou R¹R²N ;
Z est un NC, R¹O , R¹OC(O), ou R¹OCH₂CH₂(OCH₂CH₂)ₚ ;
m est égal à 0 ou 1 ; et
R¹ et R² sont indépendamment un H, un méthyle ou éthyle.

3. Procédé selon la revendication 1, dans lequel :
X est un Cl et R est un -CH₃, - (CH₂) ₂CH₃ , - (CH₂) ₃CH₃, - (CH₂) ₅CH₃, - (CH₂) ₇CH₃ , - ( CH₂ ) ₁₁CH₃, -CH(CH₃)₂, -CH (CH₃) (CH₂) ₃CH₃, - (CH₂) ₂OH, - (CH₂) ₃OH, - (CH₂CH₂O) ₂CH₂CH₂OH, - (CH₂) ₂CO₂H, -CH₂CH₂CN, -CH₂CH=CH₂ ou CH₂C₆H₅ ;
X est un Br et R est un - (CH₂) ₃CH₃ ou -CH₂C₆H₅ ; ou
X est un I et R est un - (CH₂) ₃CH₃ ou -CH₂C₆H₅ .

4. Procédé selon la revendication 1, dans lequel le cyanodithiocarbamate de formule (I) est un cyanodithiocarbamate d'hexyl-chlorométhyle.

5. Procédé selon la revendication 1, dans lequel le substrat de cuir est une peau bleue humide, une peau blanche humide, une peau à tannage végétal ou une peau tannée à l'huile.

6. Procédé selon la revendication 3, dans lequel le substrat de cuir est une peau bleue humide, une peau blanche humide, une peau à tannage végétal ou une peau tannée à l'huile.

7. Procédé selon la revendication 4, dans lequel le substrat de cuir est une peau bleue humide, une peau blanche humide, une peau à tannage végétal ou une peau tannée à l'huile.

8. Procédé de contrôle de la croissance de micro-organismes sur une peau pendant un processus de tannage de cuir, comprenant l'étape consistant à mettre en contact une peau sensible à une croissance fongique avec une liqueur de tannage contenant un cyanodithiocarbamate de formule (I) : 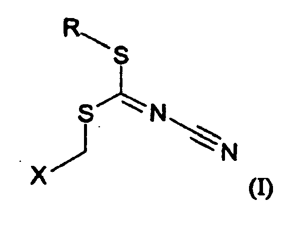
X est un halogène ;
R est un groupe alkyle en C₁-C₁₄ substitué ou non substitué, un groupe alcényle en C₂-C₁₄ substitué ou non substitué, un groupe alcynyle en C₂-C₁₄ substitué ou non substitué, un groupe substitué ou non substitué défini par Y-Ar-(CH₂)ₘ- ou par Z-(CH₂)ₙ- ;
Ar est un groupe aryle substitué ou non substitué choisi parmi le phényle et le naphthyle ;
Y est un H, un halogène, un NO₂, R¹, R¹O ou R¹R²N ;
Z est un NC, R¹O , R¹OC (O) ou R¹OCH₂CH₂ (OCH₂CH₂) ₚ ;
m est compris entre 0 et 3 ;
n est compris entre 0 et 3 ;
p est compris entre 0 et 3 ; et
R¹ et R² sont indépendamment un H, un alkyle en C₁-C₅ substitué ou non substitué ;
dans une quantité efficace pour contrôler la croissance d'au moins un champignon sur la peau tannée.

9. Procédé selon la revendication 8, dans lequel
R est un groupe alkyle en C₁-C₇ substitué ou non substitué, un groupe alcényle en C₂-C₇ substitué ou non substitué, un groupe substitué ou non substitué défini par Y-Ar-(CH₂) ₘ- ou par Z-(CH₂)ₙ- ;
Ar est un phényle ;
Y est un H, Cl, Br, I, NO₂, R¹, R¹O ou R¹R²N ;
Z est un NC, R¹O , R¹OC (O), ou R¹OCH₂CH₂ (OCH₂CH₂) ₚ ;
m est égal à 0 ou 1 ; et
R¹ et R² sont indépendamment un H, un méthyle ou éthyle.

10. Procédé selon la revendication 8, dans lequel
X est un Cl et R est un -CH₃, - (CH₂) ₂CH₃, - (CH₂) ₃CH₃, - (CH₂) ₅CH₃, - (CH₂)₇CH₃, - (CH₂) ₁₁CH₃ , -CH (CH₃) ₂, -CH (CH₃) (CH₂) ₃CH₃ , - (CH₂) ₂OH , - (CH₂) ₃OH, - (CH₂CH₂O) ₂CH₂CH₂OH, - (CH₂) ₂CO₂H, -CH₂CH₂CN, -CH₂CH=CH₂ ou CH₂C₆H₅ ;
X est un Br et R est un - (CH₂) ₃CH₃ ou -CH₂C₆H₅ ; ou
X est un I et R est un -(CH₂)₃CH₃ ou -CH₂C₆H₅.

11. Procédé selon la revendication 8, dans lequel le cyanodithiocarbamate de formule (I) est un cyanodithiocarbamate d'hexyl-chlorométhyle.

12. Procédé selon la revendication 8, dans lequel la peau est une peau bleue humide, une peau blanche humide, une peau à tannage végétal ou une peau tannée à l'huile.

13. Procédé selon la revendication 10, dans lequel la peau est une peau bleue humide, une peau blanche humide, une peau à tannage végétal ou une peau tannée à l'huile.

14. Procédé selon la revendication 11, dans lequel la peau est une peau bleue humide, une peau blanche humide, une peau à tannage végétal ou une peau tannée à l'huile.

15. Procédé selon la revendication 8, dans lequel la liqueur de tannage est une liqueur de saumure.

16. Procédé selon la revendication 8, dans lequel le cyanodithiocarbamate de formule (I) est présent dans la liqueur de tannage, dans une quantité comprise entre 5 et 500 parties par million.

17. Liqueur à utiliser dans un processus de tannage de cuir comprenant un cyanodithiocarbamate de formule (I) : 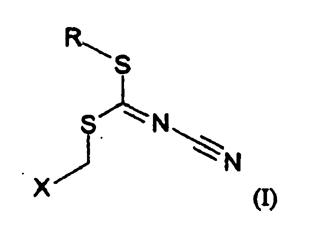
X est un halogène ;
R est un groupe alkyle en C₁-C₁₄ substitué ou non substitué, un groupe alcényle en C₂-C₁₄ substitué ou non substitué, un groupe alcynyle en C₂-C₁₄ substitué ou non substitué, un groupe substitué ou non substitué défini par Y-Ar-(CH₂)ₘ- ou par Z-(CH₂)ₙ- ;
Ar est un groupe aryle substitué ou non substitué choisi parmi le phényle et le naphthyle ;
Y est un H, un halogène, un NO₂, R¹, R¹O ou R¹R²N ;
Z est un NC, R¹O, R¹OC (O) ou R¹OCH₂CH₂(OCH₂CH₂)ₚ ;
m est compris entre 0 et 3 ;
n est compris entre 0 et 3 ;
p est compris entre 0 et 3 ; et
R¹ et R² sont indépendamment un H, un alkyle en C₁-C₅ substitué ou non substitué ;
dans laquelle la liqueur est choisie parmi une liqueur de saumure, une liqueur de tannage au chrome, une liqueur de tannage blanche, une liqueur de tannage végétal, une liqueur de tannage à l'huile, une liqueur de lavage post-tannage, une liqueur de retannage, une liqueur colorante, et une liqueur grasse ; et dans laquelle le cyanodithiocarbamate de formule (I) est présent dans la liqueur de tannage, dans une quantité comprise entre 5 et 500 parties par million.

18. Liqueur selon la revendication 17, dans laquelle
R est un groupe alkyle en C₁-C₇ substitué ou non substitué, un groupe alcényle en C₂-C₇ substitué ou non substitué, un groupe substitué ou non substitué défini par Y-Ar-(CH₂)ₘ- ou par Z-(CH₂)n- ;
Ar est un phényle ;
Y est un H, Cl, Br, I, NO₂, R¹, R¹O ou R¹R²N ;
Z est un NC, R¹O, R¹OC(O), ou R¹OCH₂CH₂(OCH₂CH₂)ₚ ;
m est égal à 0 ou 1 ; et
R¹ et R² sont indépendamment un H, un méthyle ou éthyle.

19. Liqueur selon la revendication 17, dans laquelle
X est un Cl et R est un -CH₃, - (CH₂) ₂CH₃, - (CH₂) ₃CH₃ , - (CH₂) ₅CH₃ , - (CH₂) ₇CH₃ , - (CH₂) ₁₁CH₃ , -CH (CH₃) ₂ , -CH (CH₃) (CH₂) ₃CH₃ , - (CH₂) ₂OH, - (CH₂) ₃OH, - (CH₂CH₂O) ₂CH₂CH₂OH, - (CH₂) ₂CO₂H, -CH₂CH₂CN, -CH₂CH=CH₂ ou CH₂C₆H₅ ;
X est un Br et R est un - (CH₂) ₃CH₃ ou -CH₂C₆H₅ ; ou
X est un I et R est un -(CH₂)₃CH₃ ou -CH₂C₆H₅ .

20. Liqueur selon la revendication 17, dans laquelle le cyanodithiocarbamate de formule (I) est un cyanodithiocarbamate d'hexyl-chlorométhyle.

21. Liqueur selon la revendication 17, dans laquelle la liqueur est une liqueur de saumure.

22. Liqueur selon la revendication 17, comprenant en outre un solvant choisi parmi les méthyl-éthers de glycols, le M-pyrol, et les distillats de pétrole ; et un diluant choisi parmi l'huile de soja, l'huile de pin, l'huile de semence de coton, l'huile de maïs, l'huile de colza, l'huile d'arachide, l'huile de palme, l'huile de riz, l'huile d'olive, l'huile de graine d'abrasin, l'huile de ricin, l'huile de lin, l'huile d'agrumes, et l'huile de datte.

23. Cyanodithiocarbamate de formule (I) : 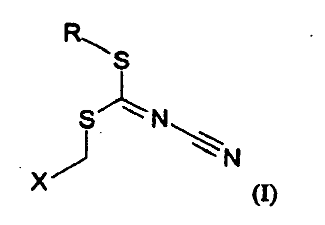
X est un halogène ;
R est un groupe alkyle en C₄-C₁₄ substitué ou non substitué, un groupe alcényle en C₂-C₁₄ substitué ou non substitué, à condition qu'il ne soit pas -CH₂CH=CH₂, un groupe alcynyle en C₂-C₁₄ substitué ou non substitué, un groupe substitué ou non substitué défini par Y-Ar-(CH₂)ₘ- ou par Z-(CH₂)ₙ- ;
Ar est un groupe aryle substitué ou non substitué choisi parmi le phényle et le naphthyle ;
Y est un H, un halogène, un NO₂, R¹, R¹O ou R¹R²N ;
Z est un NC, R¹O , R¹OC (O) ou R¹OCH₂CH₂(OCH₂CH₂)ₚ ;
m est 0, 2 ou 3 ;
n est compris entre 0 et 3 ;
p est compris entre 0 et 3 ; et
R¹ et R² sont indépendamment un H, un alkyle en C₁-C₅ substitué ou non substitué

24. Cyanodithiocarbamate selon la revendication 23, dans lequel
R est un groupe alkyle en C₅-C₇ substitué ou non substitué, un groupe alcényle en C₂-C₇ substitué ou non substitué, à condition qu'il ne soit pas -CH₂CH=CH₂, un groupe substitué ou non substitué défini par Y-Ar-(CH₂)ₘ- ou par Z-(CH₂) ₙ- ;
Ar est un phényle ;
Y est un H, Cl, Br, I, NO₂, R¹, R¹O ou R¹R²N ;
Z est un NC, R¹O, R¹OC (O) , ou R¹OCH₂CH₂(OCH₂CH₂)ₚ ;
m est égal à 0 ; et
R¹ et R² sont indépendamment un H, un méthyle ou éthyle.

25. Cyanodithiocarbamate selon la revendication 23, dans lequel
X est un Cl et R est un - (CH₂) ₅CH₃, - (CH₂) ₇CH₃, - (CH₂) ₁₁CH₃, - (CH₂) CH(CH₃)₂ , -CH(CH₃) (CH₂) ₃CH₃, -(CH₂)₂OH, -(CH₂)₃OH, -(CH₂CH₂O)₂CH₂CH₂OH, -(CH₂)₂CO2H, -CH₂CH₂CN, CH₂C₆H₅ ;
X est un Br et R est un - (CH₂) ₃CH₃ ou -CH₂C₆H₅ ; ou
X est un I et R est un - (CH₂) ₃CH₃ ou -CH₂C₆H5 .

26. Cyanodithiocarbamate d'hexyl-chlorométhyle.
